# EUROPÄISCHE PATENTSCHRIFT

(11) **EP 0 706 803 B1**
(45) Veröffentlichungstag und Bekanntmachung des Hinweises auf die Patenterteilung: **24.05.2000**
(21) Anmeldenummer: 95115436.8
(22) Anmeldetag: 29.09.1995
(51) Int. Cl.: A61L 2/26, B01F 7/00

(54) **Vorrichtung zur Unterstützung einer Gas- bzw. Dampfbewegung im Inneren einer verschliessbaren Sterilisations- bzw. Desinfektionskammer**
Device for the support of gas or vapour movement in an enclosed sterilization or disinfection chamber
Dispositif pour soutenir le mouvement de gaz ou de vapeur dans l'intérieur d'une enceinte de stérilisation ou de désinfection

(30) Priorität: 14.10.1994 DE 9416572 U
(43) Veröffentlichungstag der Anmeldung: 17.04.1996
(73) Patentinhaber: MMM MÜNCHENER MEDIZIN MECHANIK GMBH, D-81369 München (DE)
(72) Erfinder: Achterberg, Dieter, D-86949 Schöffelding (DE)
(74) Vertreter: Grünecker, Kinkeldey, Stockmair & Schwanhäusser Anwaltssozietät

(56) Entgegenhaltungen:
- EP-A- 0 487 183
- DE-U- 9 416 572
- FR-E- 90 116
- GB-A- 1 437 828

## Beschreibung

Die Erfindung bezieht sich auf eine Vorrichtung zur Erzeugung oder Unterstützung einer Gas- bzw. Dampf- oder Flüssigkeitsbewegung, insbesondere zur Ausbildung einer erzwungenen Konvektion im Inneren einer verschließbaren und mit zur Sterilisation bzw. Desinfektion vorgesehenem Gut beschickbaren Behandlungskammer, wobei über mindestens eine Fluid-Durchgangsvorrichtung Fluid zu der Behandlungskammer zuführbar und/oder aus dieser abführbar ist.

In Verbindung mit Gassterilisationsvorrichtungen zur Sterilisation mittels toxischer Gas-Mischungen sind Gas-Mischvorrichtungen bekannt, welche zur Mischung von, einer Sterilisier-Kammer separat zugeführten, Gasen jeweils mit einem in der Art eines Schaufel-Rades ausgebildeten Rührelement versehen sind. Dieses Rührelement wird über eine, durch eine abgedichtete Durchführung durch die Wand einer Sterilisierkammer hindurchgeführte Welle von einem Elektromotor angetrieben.

Alternativ zu einer derartigen Wellen-Durchführung mit gleitenden Dichtflächen ist es auch möglich, eine magnetische Kupplung zwischen dem außerhalb der Kammer liegenden Elektromotor und dem im Inneren der Kammer befindlichen Rührelement vorzusehen. Eine derartige Magnet-Kupplung erweist sich im Hinblick auf die dabei erreichbare absolute Dichtigkeit gegenüber der vorstehend genannten Vorrichtung mit einer Wellendurchführung als besonders vorteilhaft. Beide Mechanismen erweisen sichjedoch unter fertigungstechnischen Gesichtspunkten als relativ aufwendig und führen neben einer Beeinträchtigung der Festigkeit der Kammer auch zu einer Beeinträchtigung der Kammer-Isolation.

Der Erfindung liegt die Aufgabe zugrunde, eine Vorrichtung der eingangs genannten Art zu schaffen, welche einfach und preiswert herstellbar, und in zuverlässiger und wartungsarmer Weise betreibbar ist und bei Anwendung in einem Autoklaven eine Steigerung der Effektivität eines Sterilisations- bzw. Desinfektionsverfahrens auf einfache Weise ermöglicht.

Diese Aufgabe wird erfindungsgemäß durch eine Vorrichtung mit den im Patentanspruch 1 angegebenen Merkmalen gelöst.

Dadurch wird es auf vorteilhafte Weise möglich, unter Verzicht auf einen Elektromotor eine Gas-Bewegungsvorrichtung anzutreiben und das bislang ungenutzte Arbeitsvermögen des der Kammer zugeführten Fluides gezielt zur Umwälzung des in der Kammer befindlichen Fluides zu nutzen. Da der Antrieb der Vorrichtung von jenem, dem Raum zugeführten, insbesondere zur Sterilisation bzw. Desinfektion sowie anderen Verfahrens-schritten wie z.B. Trocknen vorgesehenen Fluid selbst erfolgt, wird es in vorteilhafter Weise möglich, die entsprechende Antriebsvorrichtung im Innerern des Raumes bzw. der Sterilisier- oder Desinfizierkammer anzuordnen, wodurch auf überraschend einfache Weise die vorstehend beschriebene Abdichtungsproblematik gelöst bzw. eine hohe Dichtigkeit des Systems unter Umgehung der relativ aufwendigen Magnet-Kupplung möglich wird. Neben einer Vereinfachung des schaltungstechnischen Aufwandes erweist sich die erfindungsgemäße Vorrichtung auch dahingehend als besonders vorteilhaft, als daß die Gas- oder Flüssigkeitsbewegungsvorrichtung sich gegebenenfalls selbsttätig bei Zuleitung von Fluid in den Raum bzw. zu der Kammer in Bewegung setzt. In besonders vorteilhafter Weise wird dabei das durch das Fluid bereitgestellte Arbeitsvermögen gezielt zur Unterstützung einer Gasbewegung im Inneren der Kammer herangezogen. Im Gegensatz zu den herkömmlichen Vorrichtungen verliert sich das von dem Fluid bereitgestellte Arbeitsvermögen nicht an vorgeschalteten Drosselstellen oder unmittelbar im Einleitungsbereich zu der Kammer.

Eine vorteilhafte Ausführungsform der erfindungsgemäßen Vorrichtung ist dadurch gegeben, daß die Vorrichtung zur Nutzung des Arbeitsvermögens einen druckmediumbetriebenen Motor umfaßt. Dabei ist es im allgemeinen nicht erforderlich, auf einen relativ hohen Wirkungsgrad dieser Vorrichtung abzustellen. Ein geeigneter Motor läßt sich zum Beispiel unter Verwendung eines Laufrades, das in Stanz-Biegetechnik hergestellt ist, realisieren. Zum Antrieb eignet sich in vorteilhafter Weise auch ein Motor in Flügelzellen-Bauart.

Eine zur Nutzung des durch das Fluid bereitgestellten Arbeitsvermögens besonders vorteilhafte Ausführungsform ist dadurch gegeben, daß die Vorrichtung eine Energie-Übertragungsvorrichtung in der Art einer Gebläseeinrichtung, insbesondere ein Axial-, Radial- oder Querstromgebläse, umfaßt. Dadurch wird es auf vorteilhafte Weise möglich, unter einem relativ hohen Wirkungsgrad das in der Kammer befindliche Fluid in Bewegung zu setzen bzw. in Bewegung zu halten. Dabei ist in vorteilhafter Weise die Gebläseeinrichtung mit der Vorrichtung zur Nutzung des Arbeitsvermögens antriebsverbunden.

Eine unter fertigungstechnischen Gesichtspunkten besonders vorteilhafte und preiswert realisierbare Ausführungsform der Erfindung ist dadurch gegeben, daß ein Laufrad der Gebläseeinrichtung einen von Fluid beaufschlagbaren Fluidbeaufschlagungabschnitt zum Antrieb des Laufrades unmittelbar durch das Fluid umfaßt. Ein derartiges Laufrad läßt sich auch bei einem Querstromgebläse verwirklichen. Dabei ist es möglich, entsprechende Reaktions-Abschnitte an dem Laufrad zum Beispiel durch Biegeverformung eines kleinen Flächenabschnittes einer Laufschaufel zu erhalten. Bei einer Ausführungsform der Gebläseeinrichtung in Axialbauweise kann in besonders vorteilhafter Weise an einer Stirnfläche des Axial-Laufrades eine Halb-Radialturbine vorgesehen sein. In vorteilhafter Weise ist diese Halb-Radialturbine von einem Gehäuseelement umgeben, in welchem zugleich die Lagerung des Axial-Laufrades aufgenommen ist. In vorteilhafter Weise erfolgt der Austritt des der Halb-Radialturbine zugeführten, und in der Turbine entspannten Fluides in einen Zentrumsbereich des Laufrades. Dadurch wird auf vorteilhafte Weise eine besonders effektive Verwirbelung von aus der Kammer angesaugtem Fluid mit frisch zugeführtem Fluid ermöglicht.

Eine vorteilhafte Ausführungsform der Vorrichtung ist auch dadurch gegeben, daß eine Energie-Speichereinrichtung zur Speicherung des von dem Fluid abgegebenen Arbeitsvermögens vorgesehen ist. Dadurch wird es auf vorteilhafte Weise möglich,zeitlich einem Fluid-Zuleitungsvorgang nachfolgend eine besonders effektive Bewegung bzw. Umwälzung des in der Kammer befindlichen Fluides zu gewährleisten. In vorteilhafter Weise ist dabei die Energie-Speichereinrichtung in einer Schwungmassenanordnung realisiert. Diese Schwungmassenanordnung ist in vorteilhafter Weise über eine schaltbare Kupplungsvorrichtung mit einem Laufrad der Gebläseeinrichtung verbunden. Es ist auch möglich, zwischen der Schwungmassenanordnung und dem Laufrad eine Getriebeanordnung vorzusehen zur Bereitstellung eines geeigneten Übersetzungsverhältnisses zwischen der Schwungmasse und dem Laufrad. Bei einem in der Art eines Radialgebläses ausgebildeten Laufrad ist die Schwungmassenanordnung in vorteilhafter Weise als Schwungmassen-Torus am Außenumfang des Laufrades angebracht.

Eine insbesondere im Hinblick auf eine Nachrüstung herkömmlicher Sterilisationsanlagen besonders vorteilhafte Ausführungsform der Erfindung ist dadurch gegeben, daß die Vorrichtung ein, in einem Türbereich angeordnetes Axialgebläse umfaßt. Dadurch wird es auf vorteilhafte Weise möglich, ein entsprechend einfach auswechselbares Teil der Sterilisationsanlage als vollständig vorgefertigte Baugruppe zu bevorraten. Da durch eine entsprechende Ausgestaltung der Türe eine äußerst platzsparende Anordnung des Axialgebläses möglich wird, wird das Fassungsvermögen einer entsprechend nachgerüsteten Sterilisier-Vorrichtung nicht beeinträchtigt. In vorteilhafter Weise ist es auch möglich, insbesondere im Inneren der Türe entsprechende Ansaugkanäle vorzusehen, über welche ein besonders günstiges Ansaugen von bereits in der Sterilisier-Kammer befindlichem Gas von, einer dem Fluid-Zuleitungsort relativ weit entfernten Stelle, möglich wird.

Alternativ zu oder in Kombination mit den vorstehend beschriebenen Vorrichtungen ist es auch möglich, daß die Vorrichtung mindestens ein Misch-Düsenelement umfaßt, wobei das Misch-Düsenelement einen Fluid-Beschleunigungsabschnitt und einen Fluid-Ansaugabschnitt zum Ansaugen eines bereits in der Kammer befindlichen Mediums umfaßt. Dadurch wird es möglich, die Unterstützung der Gasbewegung im Inneren der Kammer unter Verzicht auf bewegbare Teile zu realisieren. Ein derartiges Misch-Düsenelement läßt sich zum Beispiel in vorteilhafter Weise in der Art eines Eck-Leistenelementes verwirklichen. Das Eck-Leistenelement übernimmt dabei sowohl eine Fluid-Leitungsfunktion zur Leitung von, der Kammer neu zugeführtem, Fluid als auch eine Ansaugfunktion zum Ansaugen von bereits in der Kammer befindlichem Fluid. In vorteilhafter Weise umfaßt das Misch-Düsenelement einen Mischabschnitt zum Mischen des beschleunigten Fluides mit dem aus der Kammer angesaugten Medium.

Der Mischdüsenabschnitt umfaßt dabei in vorteilhafter Weise geringfügig schräg angestellte Leitbleche zur Erzeugung von lokalen Turbulenzen zur Unterstützung des Mischvorganges von angesaugtem und neu zugeführtem Fluid.

Eine vorteilhafte Ausführungsform des Misch-Düsenelementes ist auch dadurch gegeben, daß das Misch-Düsenelement einen Fluid Gemischaustrittsabschnitt umfaßt zur Abgabe einer Fluid-Mischung von aus der Kammer angesaugtem Fluid und dem der Kammer zugeführten Fluid. Dieser Fluid-Gemischaustrittsabschnitt läßt sich in vorteilhafter Weise in einem Zwischenbereich zwischen einer Wandung der Kammer und einem Leit-Blech zum Ansaugen des Fluides realisieren.

In vorteilhafter Weise ist die Vorrichtung mit einer Heiz-Einrichtung versehen. Dadurch wird es im Hinblick auf die relativ hohen Teilchen-Geschwindigkeiten im Inneren der Kammer möglich, eine relativ gleichmäßige Erwärmung bzw. Nacherhitzung des in der Kammer befindlichen Fluides, insbesondere zur Kompensation von Wärmeenergieverlusten durchzuführen. Durch die erfindungsgemäß vorgesehene Vorrichtung zur Unterstützung der Fluidbewegung wird in vorteilhafter Weise eine Überhitzung des Fluides im Bereich der Heiz-Einrichtung vermieden. Aufgrund der relativ hohen Teilchen-Geschwindigkeiten des Fluides wird zudem eine gleichmäßige Erwärmung des in der Kammer befindlichen Gutes gewährleistet.

Weitere Merkmale und Einzelheiten der Erfindung ergeben sich aus der nachfolgenden Beschreibung mehrerer Ausführungsbeispiele in Verbindung mit der Zeichnung. Es zeigen:
- Fig. 1: eine perspektivische Ansicht einer erfindungsgemäßen Vorrichtung mit einer als Querstromgebläses ausge bildeten Gebläseeinrichtung, wobei an einem stirnseitigen Ende des entsprechenden Laufrades eine, von dem der Sterilisierkammer zugeführten Fluid betriebene Antriebsvorrichtung vorgesehen ist,
- Fig.2: eine vereinfachte Darstellung einer weitere Ausführungsform einer erfindungsgemäßen Vorrichtung mit einem in Radialbauweise ausgeführten Laufrad,
- Fig.3: eine vereinfachte Darstellung einer Düsenanordnung, wie sie beispielsweise nachträglich im Eckbereich eines herkömmlichen Autoklaven einsetzbar ist,
- Fig.4: eine perspektivische Ansicht einer weiteren Ausführungsform einer Düsenanordnung mit einem in Tiefziehbauweise gefertigten Düsenelement,
- Fig.5: eine perspektivische Ansicht eines Laufrades eines Querstromgebläses mit einem mittig angeordneten Fluidbeaufschlagungsabschnitt,
- Fig.6: eine vereinfachte Darstellung zur Verdeutlichung einer bevorzugten Anordnung eines Querstromgebläses,
- Fig.7.: eine vereinfachte Darstellung eines Ausführungsbeispieles bei welchem, zur Erzeugung bzw. Unterstützung der Umwälzbewegung, ein Strahlpumpen-Prinzip Anwendung findet.

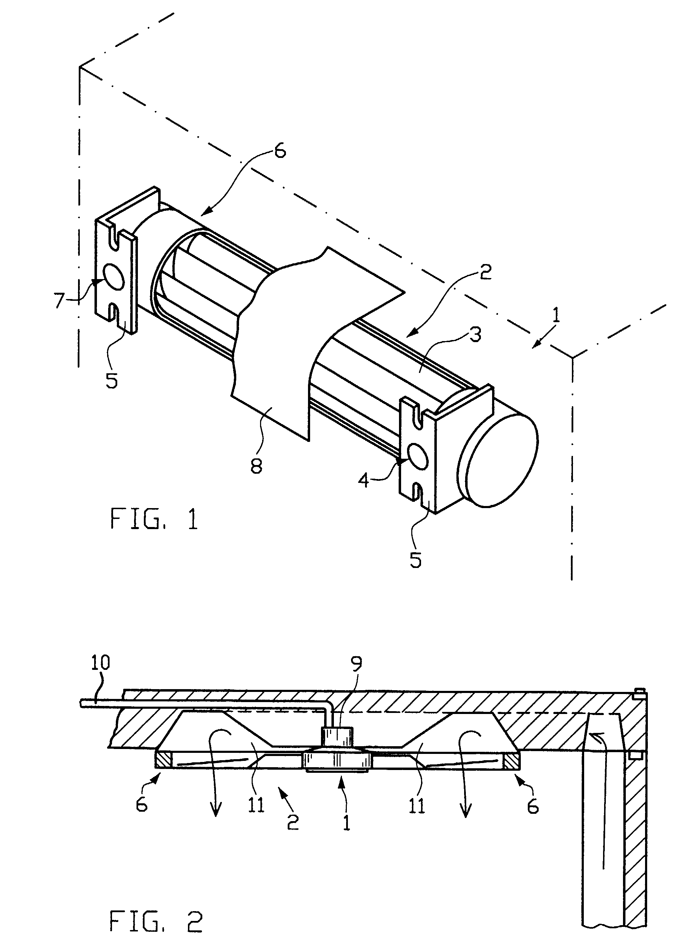

In Fig. 1 ist eine allgemein mit 1 bezeichnete Antriebsvorrichtung mit einem in Querstromgebläse-Bauart ausgeführten Laufrad 2 antriebsverbunden. Das Laufrad 2 ist mit Laufschaufeln 3 versehen, welche stirnseitig an entsprechenden Befestigungsscheiben angebracht sind. Ein zum Antrieb der Antriebsvorrichtung vorgesehenes Fluid wird über einen Fluid-Zuleitungskanal 4 der Antriebsvorrichtung 1 zugeführt. Das über den Fluid-Zuleitungskanal 4 zugeführte Fluid beaufschlagt ein im Inneren der Antriebsvorrichtung 1 vorgesehenes Turbinenlaufrad, das mit dem Laufrad 2 starr gekoppelt ist. Der Austritt des Fluides aus der Turbine erfolgt in einen Zentral-Bereich des Laufrades 2. Die Antriebsvorrichtung 1 und das Laufrad 2 sind über eine Befestigungsvorrichtung 5 mit der Wandung der Kammer verbunden. Obgleich in der bevorzugten Ausführungsform eine Fluidzufuhr zu der Antriebsvorrichtung 1 durch den Befestigungsbereich der Vorrichtung erfolgt, ist es nicht erforderlich, zwischen der Befestigungsvorrichtung 5 und dem entsprechenden Wandungsabschnitt eine Dichtvorrichtung vorzusehen, da gegebenenfalls in diesem Übergangsbereich austretendes Fluid lediglich unter Umgehung der Arbeitsmaschine der Kammer zuströmt.

Auf einer der Antriebsvorrichtung 1 gegenüberliegenden Seite des Laufrades 2 ist eine Schwungmassenanordnung 6 vorgesehen. Die Schwungmassenanordnung 6 ist über eine Kupplungsvorrichtung mit dem Laufrad 2 gekoppelt. Über einen Steuerkanal 7 ist die Schwungmassenanordnung mit dem Laufrad 2 schaltbar koppelbar. Gemäß einer bevorzugten Ausführungsform sind sowohl die Schwungmassenanordnung als auch das Laufrad 2 durch Druckbeaufschlagung des Steuerkanales 7 abbremsbar. Zur Unterstützung der Förderwirkung des in Axialbauweise ausgeführten Laufrades 2 ist mindestens ein Leit-Blech 8 vorgesehen.

Sämtliche Bauteile der vorstehend beschriebenen Vorrichtung sind in vorteilhafter Weise aus korrosionsbeständigen Materialien gefertigt. Da die Vorrichtung vollständig in dem von den zur Sterilisation bzw. Desinfektion Fluiden erfaßten System angeordnet ist, kann auf die Verwendung von entsprechenden Dicht-Vorrichtungen verzichtet werden.

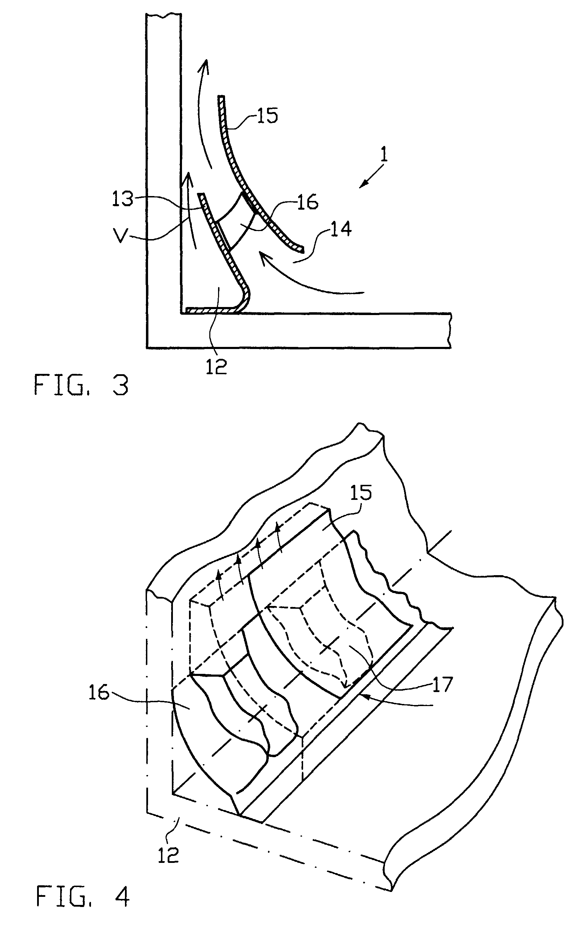

In Fig. 2 ist eine weitere Ausführungsform der erfindungsgemäßen Vorrichtung dargestellt. Dabei ist das Laufrad 2 in Axialbauweise verwirklicht. Das Laufrad 2 ist über die Antriebsvorrichtung 1 mit einem Gehäuseabschnitt der Sterilisierkammer verbunden. In den Gehäuseabschnitt ist ein Anschlußelement 9 eingesetzt und insbesondere durch eine Lötstelle mit diesem verbunden. Das Anschlußelement 9 umfaßt einen Fluid-Zuleitungsbereich und einen im wesentlichen ringförmigen Fluid-Übergangsbereich. Im Zentrum des ringförmigen Fluid-Übergangsbereiches ist eine Gewindebohrung vorgesehen, in welcher eine zentral durch die Antriebsvorrichtung 1 hindurch geführte Schraube angebracht ist. Ein FluidZuleitungsrohr 10 ist ebenfalls durch Lötung mit dem Fluid-Zuleitungselement 9 verbunden. Falls auf den Einsatz der erfindungsgemäßen Vorrichtung zunächst verzichtet werden soll, ist es möglich, lediglich das Fluid-Zuleitungselement 9 vorzusehen, wodurch optional ein nachträgliches Anbringen der erfindungsgemäßen Vorrichtung ohne besondere Änderungen an der Sterilisierkammer vorgenommen werden kann. Die Antriebsvorrichtung 1 besteht in dieser bevorzugten Ausführungsform aus einem Flügelzellen-Motor mit zwei Expansionskammern. Der Austritt des dem Motor zugeführten Fluides erfolgt aus einem unteren Stirnflächenabschnitt der Antriebsvorrichtung 1.

In einem Außenbereich des Laufrades 2 ist eine Schwungmassenanordnung 6 vorgesehen. In dieser Ausführungsform ist die Schwungmassenanordnung 6 starr mit dem Laufrad 2 verbunden. In vorteilhafter Weise erfolgt das Ansaugen von bereits in der Kammer befindlichem Fluid über Fluid-Ansaugkanäle 11. Diese Fluid-Ansaugkanäle 11 sind in vorteilhafter Weise durch Tiefziehen eines Wandungsabschnittes der Sterilisierkammer, insbesondere deren Türe, ausgebildet. Das Ansaugen des bereits in der Sterilisierkammer befindlichen Fluides erfolgt in vorteilhafter Weise aus einem der Antriebsvorrichtung 1 entfernten Bereich der Sterilisierkammer. Obgleich bei jener in Fig. 2 dargestellten Ausführungsform grundsätzlich ein Fluidstrom aus den Kanälen 11 in die Sterilisierkammer vorgesehen ist, ist es auch möglich, das Laufrad 2 zum Beispiel im Gegensinn zu betreiben und einen Fluidstrom aus der Kammer in die Kanäle 11 zu erzeugen. Das den Kanälen 11 zugeführte Fluid kann dann in vorteilhafter Weise ebenso wie in Fig. 3 dargestellt einem Misch-Düsenelement oder einer beliebigen anderen Fluid-Leitungsvorrichtung zugeführt werden.

Gemäß Fig. 3 wird die erfindungsgemäße Vorrichtung unter Verzicht auf bewegbare Teile verwirklicht. Eine Zuleitung von insbesondere "Frisch-Fluid" erfolgt in einen Druckkanal 12. Diesem Druckkanal 12 entströmt das Fluid über einen Beschleunigungsbereich 13 und saugt dabei über einen Ansaugbereich 14 ein, aus der Sterilisier- bzw. Desinfizierkammer zuströmendes, Fluid an. Das aus dem Beschleunigungsbereich 13 und dem Ansaugbereich 14 strömende Fluid vermischt sich in einem zwischen einer Wandung der Sterilisierkammer und einem Leit-Blech 15 definierten Mischbereich. Das Leit-Blech 15 ist mit einer Wandung der Druckkanales 12 über einen Abstandshalter 16 verbunden. Der Abstandshalter 16 ist in vorteilhafter Weise geringfügig zur Zuströmrichtung von aus der Kammer zuströmendem Fluid angestellt und begünstigt dadurch die Wirbelbildung zwischen dem frisch zugeführten Fluid und dem angesaugten Fluid. In vorteilhafter Weise läßt sich die in Fig. 3 dargestellte Misch Düsenanordnung auch in Kombination mit den vorstehend beschriebenen Vorrichtungen, insbesondere den Vorrichtungen gemäß Fig.1 und 2, verwenden.

Eine unter fertigungstechnischen Gesichtspunkten besonders vorteilhafte Ausführungsform des Misch-Düsenelementes ist in Fig. 4 dargestellt. Dabei ist die Wandung des Druckkanales 12 durch ein Tiefzieh-Teil 16 ausgebildet. In diesem Tiefziehteil 16 sind sowohl Fluid-Beschleunigungsabschnitte als auch Fluid-Ansaugabschnitte definiert. Das Tiefziehteil 16 ist an Verbindungsstellen 17 mit dem Leitblech 15 verbunden. Das derartig ausgebildete Misch-Düsenelement ist in einem Kantenbereich der Kammer angebracht. Je nach Erfordernis, insbesondere im Hinblick auf den Füllungsgrad ist es möglich, nur bestimmte Misch-Düsenelemente in Betrieb zu nehmen.

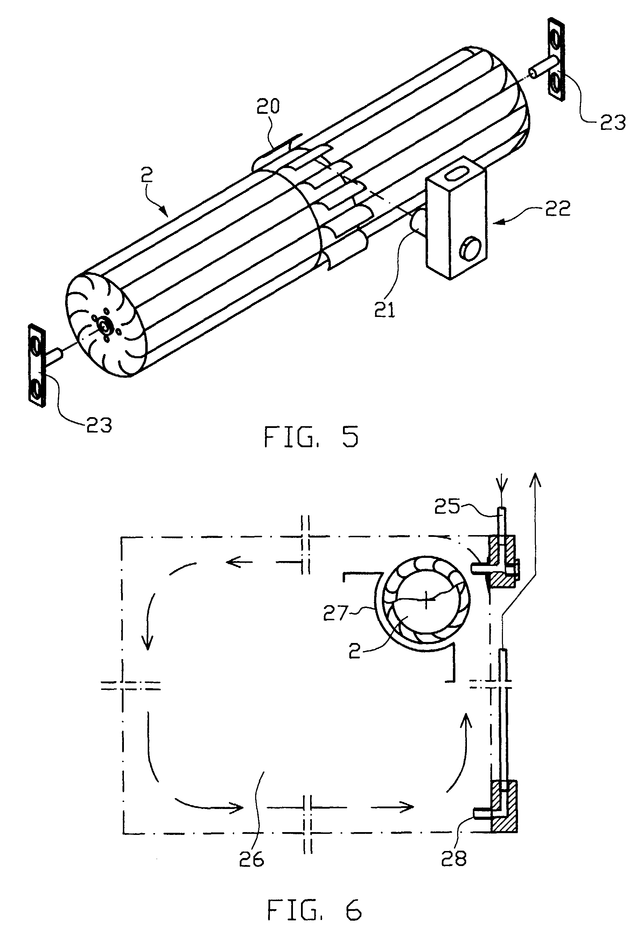

In Fig. 5 ist eine weitere bevorzugte Ausführungsform eines Laufrades eines Querstromgebläses dargestellt. Das Laufrad 2 ist hier etwa auf halber Länge mit einem Fluidbeaufschlagungsabschnitt 20 versehen, welcher starr mit dem Laufrad 2 verbunden ist. Der Fluidbeaufschlagungsabschnitt 20 wird von einem Fluidstrahl, welcher von einer Düse 21 erzeugt wird, beaufschlagt. Die Düse 21 bildet Teil eines Fluideinleitungsblockes 22 welcher ebenso wie ein Paar von Lagerungsbolzen 23 fest an der Kammer angebracht ist.

Bei der in Figur 6 dargestellten Ausführungsform der erfindungsgemäßen Vorrichtung findet ebenfalls ein in Querstromgebläse-Bauart ausgeführtes Laufrad 2 Anwendung welches ebenfalls in etwa auf halber Länge mit einem Fluidbeaufschlagungsabschnitt 20 versehen ist. Durch ein Fluidzuleitungsrohr 25 tritt ein prozeßverträgliches Fluid in die Kammer ein, wobei sich ein Strahl ausbildet, welcher auf Schaufeln eines Turbinenlaufrades trifft und dabei das Turbinenlaufrad als auch das achsgleich mit dem Turbinenlaufrad angeordnete Laufrad 2 des Querstromgebläses in Bewegung setzt. Das Querstromgebläse bewirkt im Inneren eines Raumes 26 eine Fluidumwälzung, wobei der Wirkungsgrad des Querstromgebläses durch Leitbleche 27 verbessert wird. Im Falle, daß die Fluidumwäzung nach Abschluß eines Fluideinströmvorganges länger aufrechterhalten werden soll, können durch einen Auslaßstutzen 23 bestimmte Fluidmengen aus dem Inneren des Behandlungsraumes 26 abgelassen werden.

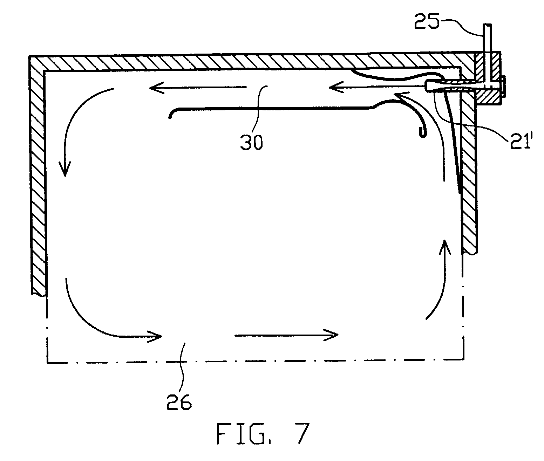

Alternativ zu oder in Kombination mit den vorangehend beschriebenen Ausführungsformen ist es wie in Figur 7 dargestellt auch möglich im Inneren der Vorrichtung eine nach dem Strahlpumpenprinzip arbeitende Umwälz-Vorrichtung zu verwenden. Bei jener in Figur 7 dargestellten Ausführungsform tritt über ein Fluidzuleitungsrohr 25 das prozeßverträgliche Fluid in eine Düse 21' in welcher das zugeführte Fluid beschleunigt wird. Durch einen aus der Düse austretenden Fluidstrahl bildet sich ein Strömungsfeld aus, in welchem ein den Fluidstrahl umgebendes Fluid beschleunigt wird. Ein der Düse 21' nachfolgend angeordneter Kanal 30, leitet ein Gemisch aus, der Kammer zugeführten und einem aus der Kammer angesaugten Prozeßfluid derart in den Behandlungsraum, daß eine durch das Arbeitsvermögen des der Kammer zugeführten Fluides unterstützte Umwälzung des Fluides in der Kammer erreicht wird.

Gemäß einer zur Sterilisation bzw. Desinfektion von Flüssigkeiten bevorzugten Ausführungsform ist eine Flüssigkeitsumwälzeinrichtung beispielsweise ein Quirl, ein Rütteltisch oder eine, in Rotationsbewegung versetzbare Tonne mit der Vorrichtung zur Nutzung mindestens eines Teiles des Arbeitsvermögens eines, der Sterilisations- bzw. Desinfektionskammer zuströmenden Fluides, antriebsverbunden. In vorteilhafter Weise wird gemeinsam mit jener in der Sterilisierkammer vorgesehenen Flüssigkeit auch das, ein oder mehrere zur Aufnahme der Flüssigkeit vorgesehene Gebinde, umgebende, in der Kammer befindliche gas- oder dampfförmige Fluid umgewälzt. Dies kann in vorteilhafter Weise dadurch erfolgen, daß zum Beispiel auf einer, zum Antrieb des Quirls vorgesehenen, Verbindungswelle ein Lüfterrad angeordnet ist. In vorteilhafter Weise ist es auch möglich, die Vorrichtung zur Nutzung mindestens eines Teiles des Arbeitsvermögens jenes der Sterilisierkammer zugeführten oder aus dieser abgeführten Fluides, mit einem Anschlußabschnitt zu versehen, an welchem wahlweise eine Gebläseeinrichtung und/oder eine Antriebswelle zum Antrieb einer Flüssigkeitsbewegungseinrichtung anbringbar ist. Dadurch wird es auf vorteilhafte Weise möglich, im Inneren einer zur Sterilisation bzw. Desinfektion vorgesehenen Kammer sowohl eine eine effektive Behandlung von Feststoffen als auch von Flüssigkeiten auf einfache Weise zu ermöglichen.

Die vorstehend beschriebene Vorrichtung läßt sich beispielsweise in nachfolgend näher beschriebener Weise verwenden. Zur Sterilisation, von zur Abpackung von Impfstoff vorgesehenen Behältnissen werden diese in entsprechende, stapelbare Aufnahmekästen eingesteckt. Die Aufnahmekästen werden in der Art eines Turmes aufeinander gestapelt und einer geöffneten Sterilisierkammer zugeführt. Die Sterilisierkammer wird geschlossen. Im Zuge eines Druckabsenkungsvorganges wird der Druck im Inneren der Sterilisierkammer vermindert. Nach Erreichen eines vorbestimmten reduzierten Druckwertes wird Fluid über einen Flügelzellen-Motor der Sterilisierkammer zugeführt. Dabei wird eine im Türbereich der Sterilisierkammer angeordnete GleichdruckFördereinrichtung angetrieben. Die Gebläseeinrichtung saugt aus einem oberen Bereich der Sterilisierkammer ein in der Sterilisierkammer befindliches Fluid an, vermengt dieses mit dem aus dem Flügelzellen-Motor austretenden Fluid und gibt einen Teil dieses Fluidgemisches an eine Misch-Düsenanordnung und an einen im wesentlichen zentral unter das Sterilisiergut führenden Kanal ab. In den Misch-Düsenelementen wird durch das zugeführte Fluid erneut bereits in der Kammer befindliches Fluid angesaugt und umgewälzt. Nach Abschluß des Fluidzuleitungsvorganges wird über eine Schwungmassenanordnung über einen längeren Zeitabschnitt die Fluidbewegung im Inneren der Kammer unterstützt. Zur erneuten Inbetriebnahme der Gebläseeinrichtung ist es möglich, parallel zur Fluid-Zufuhr, Fluid aus der Sterilisierkammer abzuleiten. Gegen Ende des Fluid-Umwälzvorganges im Inneren der Sterilisierkammer wird der Kammer eine Gas-Probe entnommen und diese im Hinblick auf ihre Zusammensetzung analysiert. Im Rahmen eines nachfolgenden Druckabsenkungsschrittes wird der Druck im Inneren der Kammer erneut reduziert. Im Rahmen eines darauf folgenden weiteren Verfahrensschrittes wird wiederholt Fluid zu der Sterilisierkammer unter Inbetriebnahme der Gleichdruck-Fördereinrichtung zugeführt. Nach Abschluß dieses zweiten Sterilisierschrittes wird die Antriebsvorrichtung zum Betrieb der Gebläseeinrichtung derart geschaltet, daß die Gebläseeinrichtung durch, aus der Sterilisierkammer abstretendes.Fluid angetrieben wird. Bei Erreichen eines bestimmten Druckwertes wird der Kammer ein Trocken- oder Spülfluid, zum Trocknen oder Spülen, über die Vorrichtung zur Nutzung des Arbeitsvermögens zugeführt und dabei gleichzeitig die Gleichdruck-Fördereinrichtung erneut in Betrieb gesetzt. Unter Zuströmung des Fluides und bei gleichzeitigem Betrieb der Gebläseeinrichtung wird ein besonders effektiver Spül- oder Trocknungsvorgang gewährleistet. Im Anschluß an diesen Verfahrensschritt wird ein Druckausgleich zwischen der Sterilisierkammer und der Umgebung der Kammer hergestellt. Nach Druckausgleich wird die Sterilisierkammer geöffnet und das nunmehr sterilisierte Sterilisiergut der Kammer entnommen und dem weiteren Verwendungszweck zugeführt.

Die Erfindung ist nicht auf das vorstehend beschriebene Ausführungsbeispiel beschränkt, beispielsweise ist das vorstehend beschriebene Misch-Düsenelement auch ohne der genannten Gebläseeinrichtung anwendbar.

## Patentansprüche

1. Vorrichtung zur Erzeugung oder Unterstützung einer Fluidbewegung im Inneren einer verschließbaren Behandlungskammer, wobei diese Behandlungskammer mit Gut, das in der Behandlungskammer einer Sterilisations- oder Desinfektionsbehandlung unterzogen wird, beschickbar ist, und über mindestens eine Fluid-Durchgangsvorrichtung der Behandlungskammer ein Fluid zuführbar und/oder aus dieser abführbar ist, wobei eine Einrichtung zur Nutzung mindestens eines Teils des Arbeitsvermögens des zuströmenden oder abströmenden Fluides zur Erzeugung oder Unterstützung einer Umwälzbewegung eines in der Behandlungskammer befindlichen gas-bzw. dampfförmigen oder flüssigen Mediums durch das Fluid vorgesehen ist.

2. Vorrichtung nach Anspruch 1, **dadurch gekennzeichnet,** daß die Vorrichtung zur Nutzung des Arbeitsvermögens des Fluides als Druckmedium betriebener Motor ausgebildet ist.

3. Vorrichtung nach Anspruch 1 oder 2, **dadurch gekennzeichnet,** daß die Vorrichtung eine Energieübertragungsvorrichtung in der Art einer Gebläseeinrichtung, insbesondere ein Axial-, Radial- oder Querstromgebläse, umfaßt.

4. Vorrichtung nach mindestens einem der Ansprüche 1 bis 3, **dadurch gekennzeichnet,** daß die Gebläseeinrichtung mit der Vorrichtung zur Nutzung des Arbeitsvermögens des Fluides antriebsverbunden ist.

5. Vorrichtung nach mindestens einem der Ansprüche 1 bis 4, **dadurch gekennzeichnet,** daß ein Laufrad der Gebläseeinrichtung einen von Fluid beaufschlagbaren Fluidbeaufschlagungsabschnitt zum Antrieb des Laufrades unmittelbar durch das Fluid umfaßt.

6. Vorrichtung nach mindestens einem der Ansprüche 1 bis 5, **dadurch gekennzeichnet,** daß eine Energiespeichereinrichtung zur Speicherung eines von dem Fluid abgeleisteten Arbeitsvermögens vorgesehen ist.

7. Vorrichtung nach mindestens einem der Ansprüche 1 bis 6, **dadurch gekennzeichnet,** daß die Energiespeichereinrichtung eine Schwungmassenanordnung umfaßt.

8. Vorrichtung nach mindestens einem der Ansprüche 1 bis 7, **dadurch gekennzeichnet,** daß die Gebläseeinrichtung in einem Türbereich angeordnet, insbesondere an einer Türe befestigt ist.

9. Vorrichtung nach mindestens einem der Ansprüche 1 bis 8, **dadurch gekennzeichnet,** daß die Einrichtung zur Nutzung des Arbeitsvermögens mindestens ein Mischdüsenelement umfaßt, wobei das Mischdüsenelement einen Fluidbeschleunigungsabschnitt und einen Fluidansaugabschnitt zum Ansaugen eines bereits in der Kammer befindlichen Mediums umfaßt.

10. Vorrichtung nach mindestens einem der Ansprüche 1 bis 9, **dadurch gekennzeichnet,** daß das Mischdüsenelement einen Mischabschnitt umfaßt zum Mischen des beschleunigten Fluides mit dem aus der Kammer angesaugten Medium.

11. Vorrichtung nach mindestens einem der Ansprüche 1 bis 10, **dadurch gekennzeichnet,** daß das Misch-Düsenelement einen Fluid-Gemischaustrittsabschnitt umfaßt zur Abgabe einer Fluidmischung von aus der Kammer angesaugten Fluid und dem der Kammer zugeführten Fluid.

12. Vorrichtung nach mindestens einem der Ansprüche 1 bis 11, **dadurch gekennzeichnet,** daß das angesaugte bzw. umgewälzte Fluid in seiner chemischen Zusammensetzung dem zugeführten Fluid gleicht.

13. Vorrichtung nach mindestens einem der Ansprüche 1 bis 12, **dadurch gekennzeichnet,** daß mehrere Fluidbeschleunigungsdüsen vorgesehen sind.

14. Vorrichtung nach mindestens einem der Ansprüche 1 bis 13, **dadurch gekennzeichnet,** daß mindestens eine der Fluidbeschleunigungsdüsen einen spaltförmigen Querschnitt aufweist.

15. Vorrichtung nach mindestens einem der Ansprüche 2 bis 14, **dadurch gekennzeichnet,** daß die Vorrichtungen gemäß Patentanspruch 2 und Patentanspruch 9 in Kombination vorgesehen sind.

## Claims

1. Device for producing or supporting a fluid movement in the interior of a sealable treatment chamber, wherein this treatment chamber can be charged with material which is subjected in the treatment chamber to a sterilisation or disinfection treatment, and a fluid can be supplied to the treatment chamber and/or removed from the latter via at least one fluid pass-through device, wherein an appliance for utilising at least some of the potential energy of the inflowing or outflowing fluid is provided to produce or support a circulating movement of a gaseous or vaporous or liquid medium, contained in the treatment chamber, through the fluid.

2. Device according to claim 1, characterised in that the device for utilising the potential energy of the fluid is formed as a pressure medium-operated motor.

3. Device according to claim 1 or 2, characterised in that the device comprises an energy transmission device in the manner of a blower appliance, in particular an axial-, a radial- or a cross-flow blower.

4. Device according to at least one of claims 1 to 3, characterised in that the blower appliance is connected in drive terms to the device for utilising the potential energy of the fluid.

5. Device according to at least one of claims 1 to 4, characterised in that an impeller of the blower appliance comprises a fluid impact section on which fluid can act for driving the impeller directly through the fluid.

6. Device according to at least one of claims 1 to 5, characterised in that an energy storage device is provided to store potential energy derived from the fluid.

7. Device according to at least one of claims 1 to 6, characterised in that the energy storage device comprises an inertial mass arrangement.

8. Device according to at least one of claims 1 to 7, characterised in that the blower appliance is disposed in a door region, being in particular attached to a door.

9. Device according to at least one of claims 1 to 8, characterised in that the appliance for utilising the potential energy comprises at least one mixing nozzle element, wherein the mixing nozzle element comprises a fluid accelerating section and a fluid intake section for taking in a medium already contained in the chamber.

10. Device according to at least one of claims 1 to 9, characterised in that the mixing nozzle element comprises a mixing section for mixing the accelerated fluid with the medium taken in from the chamber.

11. Device according to at least one of claims 1 to 10, characterised in that the mixing nozzle element comprises a fluid mixture outlet section for delivering a fluid mixture of fluid taken in from the chamber and the fluid supplied to the chamber.

12. Device according to at least one of claims 1 to 11, characterised in that the fluid taken in or circulated is equal to the supplied fluid in terms of its chemical composition.

13. Device according to at least one of claims 1 to 12, characterised in that a plurality of fluid accelerating nozzles are provided.

14. Device according to at least one of claims 1 to 13, characterised in that at least one of the fluid accelerating nozzles has a slit-shaped cross section.

15. Device according to at least one of claims 2 to 14, characterised in that the devices according to claim 2 and claim 9 are provided in combination.

## Revendications

1. Dispositif pour produire ou soutenir le déplacement d'un fluide à l'intérieur d'une chambre de traitement pouvant être fermée, cette chambre de traitement pouvant être alimentée avec un matériau qui est soumis dans la chambre de traitement à un traitement de stérilisation ou de désinfection, et un fluide pouvant être amené dans la chambre de traitement et/ou évacué de celle-ci par au moins un dispositif de passage de fluide, dans lequel un dispositif est prévu pour utiliser au moins une partie de l'énergie du fluide amené ou évacué pour produire ou soutenir à l'aide de ce fluide la circulation d'un milieu gazeux ou à l'état de vapeur ou liquide se trouvant dans la chambre de traitement.

2. Dispositif selon la revendication 1, caractérisé en ce que le dispositif pour utiliser l'énergie du fluide est réalisé sous la forme d'un moteur actionné par un fluide sous pression.

3. Dispositif selon la revendication 1 ou 2, caractérisé en ce que le dispositif comprend un dispositif de transport d'énergie sous la forme d'un dispositif à ventilateur, en particulier d'un ventilateur à courant axial, radial ou transversal.

4. Dispositif selon au moins une des revendications 1 à 3, caractérisé en ce que le dispositif à ventilateur est lié du point de vue de l'entraînement au dispositif pour utiliser l'énergie du fluide.

5. Dispositif selon au moins une des revendications 1 à 4, caractérisé en ce qu'une roue du dispositif à ventilateur comprend un segment pouvant être parcouru par un fluide afin d'entraîner la roue directement par le fluide.

6. Dispositif selon au moins une des revendications 1 à 5, caractérisé en ce qu'un dispositif d'accumulation d'énergie est prévu pour accumuler une énergie produite par le fluide.

7. Dispositif selon au moins une des revendications 1 à 6, caractérisé en ce que le dispositif d'accumulation d'énergie comprend un agencement à masse mobile.

8. Dispositif selon au moins une des revendications 1 à 7, caractérisé en ce que le dispositif à ventilateur est disposé dans la région d'une porte, et est notamment fixé sur une porte.

9. Dispositif selon au moins une des revendications 1 à 8, caractérisé en ce que le dispositif pour utiliser l'énergie comprend au moins un élément à buse mélangeuse, l'élément à buse mélangeuse comprenant une partie d'accélération de fluide et une partie d'aspiration de fluide pour aspirer un milieu déjà présent dans la chambre.

10. Dispositif selon au moins une des revendications 1 à 9, caractérisé en ce que l'élément à buse mélangeuse comprend une partie mélangeuse pour mélanger le fluide accéléré avec le milieu aspiré dans la chambre.

11. Dispositif selon au moins une des revendications 1 à 10, caractérisé en ce que l'élément à buse mélangeuse comprend une partie de sortie de mélange de fluides destinée à évacuer un mélange de fluides formé de fluide aspiré dans la chambre et du fluide amené dans la chambre.

12. Dispositif selon au moins une des revendications 1 à 11, caractérisé en ce que le fluide aspiré ou mis en circulation a la même composition chimique que le fluide amené.

13. Dispositif selon au moins une des revendications 1 à 12, caractérisé en ce que plusieurs buses d'accélération de fluide sont prévues.

14. Dispositif selon au moins une des revendications 1 à 13, caractérisé en ce que au moins une des buses d'accélération de fluide présente une section transversale en forme de fente.

15. Dispositif selon au moins une des revendications 2 à 14, caractérisé en ce que les dispositifs selon la revendication 2 et selon la revendication 9 sont combinés.
